# EUROPEAN PATENT SPECIFICATION

(11) **EP 3 652 532 B1**
(45) Date of publication and mention of the grant of the patent: **26.06.2024**
(21) Application number: 18831181.5
(22) Date of filing: 04.07.2018
(51) Int. Cl.: C02F 1/00, E03B 1/04, E03C 1/00, E03C 1/122, G01N 1/20, G01N 33/18

(54) **OFF-LINE BYPASS LOOP ARRANGEMENT FOR A WATER RECYCLING DEVICE**
OFFLINE-BYPASS-SCHLEIFENANORDNUNG FÜR EINE WASSERRECYCLING-VORRICHTUNG
AGENCEMENT DE BOUCLE DE DÉRIVATION HORS LIGNE POUR UN DISPOSITIF DE RECYCLAGE D'EAU

(30) Priority: 14.07.2017 SE 1750925
(43) Date of publication of application: 20.05.2020
(73) Proprietor: Orbital Systems AB, 211 20 Malmö (SE)
(72) Inventor: RIDELL, Michael, 245 42 Staffanstorp (SE)
(74) Representative: AWA Sweden AB
(86) International application number: PCT/SE2018/050734
(87) International publication number: WO 2019/013691

(56) References cited:
- EP-A1- 3 045 891
- EP-A1- 3 045 891
- EP-A1- 3 755 846
- EP-A1- 3 755 847
- WO-A1-2004/101902
- WO-A1-2004/101902
- WO-A1-2015/115995
- WO-A1-2017/099663
- GB-A- 2 552 989
- US-A1- 2009 098 022
- US-A1- 2016 312 447
- US-A1- 2017 122 326

## Description

### Field of the invention

The present invention relates to a method for measuring one or more parameters in a water flow in a device intended for recycling of water, to a measuring unit intended for a water recycling device and to a water recycling device comprising such a measuring unit.

### Technical Background

There are many different types of water recirculation systems. One such is shown in WO2013/095278 A1, which describes a hybrid device for a recirculation shower, allowing purification and either recycling of water or discarding of water, wherein said hybrid device comprises a recirculation loop, a filter system with a nano-filter, at least one filter quality sensor, at least one pre-filter, and wherein the hybrid device is arranged to redirect the water from recirculation to drainage when the at least one filter quality sensor indicates the need thereof.

Furthermore, in WO2017/099663 A1 there is also disclosed a water recirculation apparatus, wherein the apparatus comprises a light unit intended for neutralisation of organisms in said water flow, and wherein the light unit, such as a UV LED unit, is arranged in a dockable water treatment chamber. Water recirculation systems are futher known from US2016/312447 A1 and WO2004/101902 A1. The present invention also relates to water recirculation systems, e.g such showers. The present invention is directed to providing an improved measuring method and a measuring unit intended for water recirculation systems.

### Summary of the invention

The stated purpose above is achieved by a method for measuring one or more parameters in a water flow in a device in the form of a recirculating shower according to independent claim 1. The concept of the present invention is to ensure that the measuring is performed on a water amount which is stagnant. This has several advantages, Firstly, as the water amount is not in movement, there is no or a very little risk of air bubbles, which in themselves is an error source for the measurement. Secondly, as the water amount is stagnant, the present invention provides the control of measuring several parameters on one and the same water amount, not only once but also during several times if this is of interest, before a specific water amount is conversed and replaced. Both advantages mentioned above are important when measuring in a system where water is flowing, such as in a water recirculation system, e.g. in a recirculating shower.

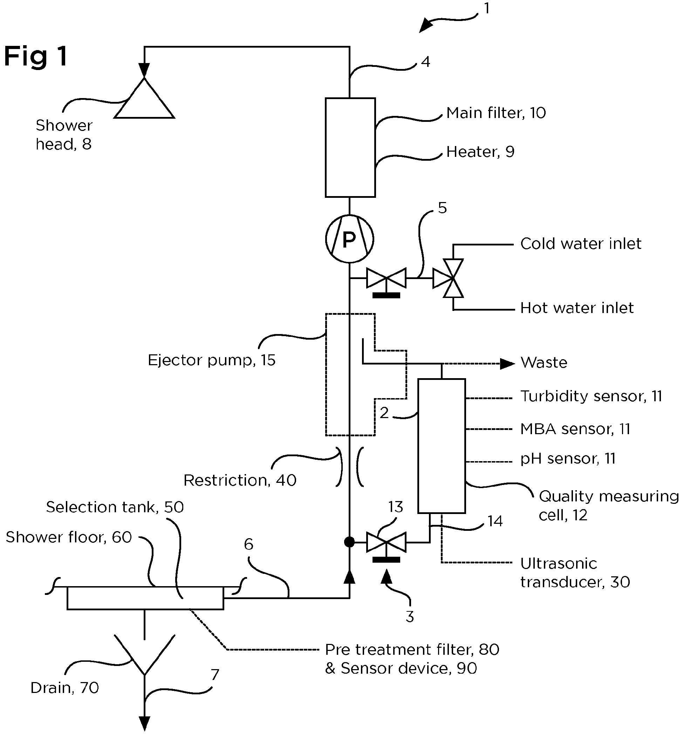

The present invention is also directed to a device in the form of a recirculating shower according to independent claim 5. One example of such a device is shown in fig. 1 and the apparatus concept of the present invention is further described below.

### Specific embodiments of the invention

Below, specific embodiments of the present invention are disclosed. According to the present invention, the liquid-stagnant place is arranged as an off-line bypass loop arrangement comprising a quality measuring cell in the device intended for recycling of water. It should be noted that the present invention covers all systems where the liquid is led to liquid-stagnant space for measurement, however an off-line bypass arrangement is one preferred alternative. This off-line bypass arrangement is further discussed below.

According to yet another specific embodiment of the present invention, the step of directing part of the water flow to a liquid-stagnant space is performed cyclically. Cyclically should in this regard be linked to the sampling timing, implying that the method according to the present invention is performed repeatedly. The actual measuring may be performed linked to a minimum off-line regulation, i.e. measuring first after a certain time range when it is ensured that the water flow is stopped. Moreover, the actual measuring frequency for different parameters may vary so that certain parameters, e.g. turbidity, may be measured every sampling cycle, however other parameters, such as microbiological activity, may be measured only once a day or so.

As may be understood from above, the method may involve measuring one or more different parameters. According to the present invention, the measuring is performed on one or more of the parameters turbidity, conductivity, pH value, ultrasound and microbiological activity. It should be noted that any combinations of parameters above are possible, and according to one specific embodiment of the present invention, measuring is performed on at least turbidity, which may be a key parameter in several cases. Turbidity is of interest to measure the water quality, and may function as a quality measurement of a primary water quality measurement performed by measuring conductivity (with EC sensor(s)). pH value indicates another important parameter for water. Ultrasound is induced to remove air bubbles, e.g. by use of an ultrasonic transducer (see fig. 1), but may also be measured by an ultrasound sensor. Measuring the ultrasound may be implemented by measuring the time for ultrasound to travel a known distance, which is also called time of flight (TOF).

Moreover, the present invention may also involve measuring other parameters not mentioned above, such as water hardness. Furthermore, the method according to the present invention may also involve adding a reagent to the liquid-stagnant space before measuring a certain parameter.

According to yet another specific embodiment of the present invention, measuring is performed on turbidity, and reference value is set as a start by measuring penetrated light through fresh water, and this is used as a reference, and wherein the turbidity then is measured by comparing liquid light penetration with reference, and as such enabling to determine water quality. This in itself is different than measuring turbidity according to the standard. According to the present invention, calibration may be performed for a water recycling device and the control system and operation method thereof during the production of the device by using what is known to be fresh water. As such, a reference value may be set, and therefore it may be enough to measure a difference to this reference value, and not an absolute value as is the standard. According to this embodiment of the present invention, the actual measurement is simplified as it is enough to measure the level of light penetrating from one side of a tank or quality measuring cell (see below) or the like containing the liquid-stagnant space.

The present invention also provides a device intended for recycling of water, where said device comprises an off-line bypass loop arrangement which comprises a liquid-stagnant space and which off-line bypass loop arrangement also comprises one or more sensors enabling measuring in the off-line bypass loop arrangement.

According to the present invention, the off-line bypass loop arrangement comprises a quality measuring cell and a magnetic valve. The actual measuring is being performed in the quality measuring cell and the magnetic valve is provided to ensure a filling of a new measuring amount after emptying the same and when a new measurement is intended.

As is shown in fig. 1, the entire off-line bypass loop arrangement may comprise a measuring tank which is arranged on a bypass line within the off-line bypass loop arrangement, and where the off-line bypass loop arrangement also comprises an ejector pump. The quality measuring cell is emptied by use of the ejector pump. The content therein is either sent up further in the recirculation loop when the water "passes" the quality measurement levels, or otherwise sent to the waste. When exchanging the liquid in the quality measuring cell again, the magnetic valve is opened. Suitably this is performed in the same sequence so that the quality measuring tank is always full, or at least not empty.

According to one embodiment of the present invention, the off-line bypass loop arrangement is arranged as a suction unit on the low pressure side of the device intended for recycling of water. One such alternative is shown in fig. 1. By using the low pressure side, the utilization sequence is simplified.

The device according to the present invention comprises one or several sensors. According to the present invention, the off-line bypass loop arrangement comprises sensors for measurement of at least one of the parameters turbidity, conductivity, pH value, ultrasound and microbiological activity. According to one specific embodiment, the off-line bypass loop arrangement comprises a turbidity sensor unit.

It should be noted that also other sensors may be arranged, e.g. for measuring the water hardness. The sensors are connected to a control system which is also connected to other sensors or data handling or sourcing units. The data may be used to analyse the incoming water as such, e.g. in the case of water hardness, but the mina purpose is for measuring the real-time water quality in a water recirculating system, such as a recirculating shower. Moreover, the data collected may also give input to decisions being made in the control system, such as with reference to if water should be recirculated or discarded or how often and when cleaning of the device should be performed etc. etc.

As is further discussed below, the off-line bypass loop arrangement may also comprise an ultrasonic transducer. This is further discussed below, also in relation to reducing the error source of air bubbles when using turbidity as a water quality parameter being measured.

### Detailed description of the drawings

In fig. 1 there is shown a device 1 intended for recycling of water, in this case a recirculating shower, where the device 1 comprises a liquid-stagnant space 2 where measuring is intended to be performed. The liquid-stagnant space 2 is positioned in an off-line bypass loop arrangement 3 of the device 1, which off-line bypass loop arrangement 3 also comprises one or more sensors 11 enabling measuring.

Moreover, the device 1 also comprises a flow path 4 for recycled water, a fresh water inlet 5 (cold and hot water inlet), a recirculation water inlet 6 from a selection tank and a recirculation water outlet 7, a user outlet 8 (shower head), a heater 9 and a main filter 10 enabling the recycling feature. As seen in fig. 1, the water flowed in via the fresh water inlet 5 (cold and hot water inlet) meets a recirculation loop comprising the recirculation water inlet 6, which in fact is one point along the loop before subsequent key units being a heater 9 and filter 10. In the flow path up-streams, the user outlet 8, in this case a shower head, is provided subsequently to the heater 9 and filter 10. As seen in fig. 1 there is arranged a selection tank 50 being a point from which water is either wasted via the water recirculation outlet (drain 70) or sent to recirculation. As the water recirculation device in this case is a recirculating shower, the selection tank is provided in the shower floor 60. The selection tank 50 suitably comprises a pre-filter or rough filter 80 and a sensor 90. This sensor enables for the system to decide if water should be discarded and sent to the drain or recycled. As seen in the flow path 4 for recycled water, there is provided a heater 9 and a main filter 10, which suitably is arranged in a combined heater and filter unit. Moreover, this unit may also comprise a water treatment source, such as a light unit, e.g. a UV lamp, to be used for killing microorganisms in the water.

Moreover, the off-line bypass loop arrangement 3 comprises a quality measuring tank 12 and a magnetic valve 13. The quality measuring tank 12 may be seen as a conversion tank, and can be a comparatively small unit, such as between 0.5 and 1.5 litres, e.g. between 0.5 and 1.0 litres, in case of recirculation shower as shown in fig. 1. As seen in fig. 1, the quality measuring tank 12 is arranged on a bypass line 14 within the off-line bypass loop arrangement 3, and where the off-line bypass loop arrangement 3 also comprises an ejector pump 15. When a new measuring cycled is to be performed the ejector pump 15 sucks water from the mail line as well as the quality measuring tank 12. The restriction 40 is provided to enable the operation of the ejector pump 15. As the magnetic valve 13 is open then also a new water conversion amount is pumped into the measuring tank 15. The measuring is then being performed on this water amount.

Several sensors 11 may be arranged in the off-line bypass loop arrangement 3, suitably in the quality measuring tank 12 as shown in fig. 1. In this case there is at least provided sensors 11 for measuring turbidity, microbiological activity (MBA) and pH value, but other ones may be provided in addition to these on instead of these. Furthermore, an ultrasonic transducer 30 may also be arranged in the quality measuring tank 12 as shown in fig. 1. This transducer 30 may be used to reduce the number or air bubbles by introducing ultrasound, and may also be used to measure the level of ultrasound being introduced. Air bubbles may affect the measurement of turbidity and therefore it may be of relevance to reduce or diminish the amount of air bubbles.

Moreover, and as shown in fig. 1, the quality measuring cell 12 is also linked to the waste so that the water amount held therein may be directly sent to the waste if needed.

## Claims

1. Method for measuring one or more parameters in a water flow in a device (1) in the form of a recirculating shower, said device (1) comprising a fresh water inlet (5) for cold and hot water, which meets a recirculation loop comprising a recirculation water inlet (6), a flow path (4) for recycled water and a recirculation water outlet (7), a user outlet (8) in the form of a shower head, a heater (9) and a filter (10) arranged along the recirculation loop, and wherein the device (1) also comprises an off-line bypass loop arrangement (3) which comprises a liquid-stagnant space (2) and which off-line bypass loop arrangement (3) also comprises one or more sensors (11) enabling measuring in the off-line bypass loop arrangement (3),
said method comprising directing part of the water flow to the liquid-stagnant space (2) where the measuring is performed,
wherein the liquid-stagnant place (2) is arranged as an off-line bypass loop arrangement (3) comprising a quality measuring cell (12) in the device (1) intended for recycling of water,
and wherein the measuring is performed on one or more of the parameters turbidity, conductivity, pH value, ultrasound and microbiological activity.

2. Method according to claim 1, wherein the step of directing part of the water flow to a liquid-stagnant space (2) is performed cyclically.

3. Method according to any of the preceding claims, wherein measuring is performed on at least turbidity.

4. Method according to any of the preceding claims, wherein measuring is performed on turbidity, wherein a reference value is set as a start by measuring penetrated light through fresh water, and this is used as a reference, and wherein the turbidity then is measured by comparing liquid light penetration with reference, and as such enabling to determine water quality.

5. Device (1) in the form of a recirculating shower, said device (1) comprising a flow path (4) for recycled water, a fresh water inlet (5) for cold and hot water, which meets a recirculation loop comprising a recirculation water inlet (6), a flow path (4) for recycled water and a recirculation water outlet (7), a user outlet (8) in the form of a shower head, a heater (9) and a filter (10) arranged along the recirculation loop,
the device (1) being **characterized in that** it also comprises an off-line bypass loop arrangement (3) which comprises a liquid-stagnant space (2) and which off-line bypass loop arrangement (3) also comprises one or more sensors (11) enabling measuring in the off-line bypass loop arrangement (3),
wherein the off-line bypass loop arrangement (3) comprises a quality measuring cell (12) and a magnetic valve (13),
and wherein the off-line bypass loop arrangement (3) comprises sensors (11) for measurement of at least one of the parameters turbidity, conductivity, pH value, ultrasound and microbiological activity.

6. Device (1) according to claim 5, wherein the measuring tank (12) is arranged on a bypass line (14) within the off-line bypass loop arrangement (3), and wherein the off-line bypass loop arrangement (3) also comprises an ejector pump (15).

7. Device (1) according to any of claims 5-6, wherein the off-line bypass loop arrangement (3) is arranged as a suction unit on the low pressure side of the device (1) intended for recycling of water.

8. Device (1) according to any of claims 5-7, wherein the off-line bypass loop arrangement (3) comprises an ultrasonic transducer (30).

9. Device (1) according to any of claims 5-8, wherein the off-line bypass loop arrangement (3) comprises a turbidity sensor unit.

## Patentansprüche

1. Verfahren zur Messung eines oder mehrerer Parameter in einem Wasserstrom in einer Vorrichtung (1) in der Form einer rezirkulierenden Dusche, wobei die Vorrichtung (1) einen Frischwassereinlass (5) für kaltes und heißes Wasser umfasst, der zu einer Rezirkulationsschleife führt, die einen Rezirkulationswassereinlass (6), einen Strömungsweg (4) für rückgeführtes Wasser und einen Rezirkulationswasserauslass (7), einen Benutzerauslass (8) in der Form eines Duschkopfs, eine Heizvorrichtung (9) und einen Filter (10) umfasst, die entlang der Rezirkulationsschleife angeordnet sind, und wobei die Vorrichtung (1) ferner eine Umgehungsschleifenanordnung im Nebenstrom (3) umfasst, die einen Raum mit stehender Flüssigkeit (2) umfasst, und wobei die Umgehungsschleifenanordnung im Nebenstrom (3) ferner einen oder mehrere Sensoren (11) umfasst, die Messung in der Umgehungsschleifenanordnung im Nebenstrom (3) ermöglichen,
wobei das Verfahren Leiten eines Teils des Wasserstroms zu dem Raum mit stehender Flüssigkeit (2) umfasst, wo die Messung durchgeführt wird,
wobei der Raum mit stehender Flüssigkeit (2) als eine Umgehungsschleifenanordnung im Nebenstrom (3) angeordnet ist, die eine Qualitätsmessungszelle (12) in der zur Rückführung von Wasser vorgesehenen Vorrichtung (1) umfasst,
und wobei die Messung für einen oder mehrere der Parameter Trübung, Leitfähigkeit, pH-Wert, Ultraschall und mikrobiologischer Aktivität durchgeführt wird.

2. Verfahren gemäß Anspruch 1, wobei der Schritt des Leitens eines Teils des Wasserstroms zu einem Raum mit stehender Flüssigkeit (2) zyklisch durchgeführt wird.

3. Verfahren gemäß einem der vorstehenden Ansprüche, wobei Messen wenigstens für Trübung durchgeführt wird.

4. Verfahren gemäß einem der vorstehenden Ansprüche, wobei Messen für Trübung durchgeführt wird, wobei ein Bezugswert als Ausgangspunkt durch Messen von durchgehendem Licht durch Frischwasser eingestellt wird und dies als Bezug verwendet wird und wobei die Trübung anschließend durch Vergleichen von durch Flüssigkeit durchgehendem Licht mit dem Bezugswert durchgeführt wird, um die Wasserqualität bestimmen zu können.

5. Vorrichtung (1) in der Form einer rezirkulierenden Dusche, wobei die Vorrichtung (1) einen Strömungsweg (4) für rückgeführtes Wasser, einen Frischwassereinlass (5) für kaltes und heißes Wasser, der zu einer Rezirkulationsschleife führt, die einen Rezirkulationswassereinlass (6), einen Strömungsweg (4) für rückgeführtes Wasser und einen Rezirkulationswasserauslass (7), einen Benutzerauslass (8) in der Form eines Duschkopfs, eine Heizvorrichtung (9) und einen Filter (10) umfasst, die entlang der Rezirkulationsschleife angeordnet sind, wobei die Vorrichtung (1) **dadurch gekennzeichnet ist, dass** sie ferner eine Umgehungsschleifenanordnung im Nebenstrom (3) umfasst, die einen Raum mit stehender Flüssigkeit (2) umfasst, und wobei die Umgehungsschleifenanordnung im Nebenstrom (3) ferner einen oder mehrere Sensoren (11) umfasst, die Messung in der Umgehungsschleifenanordnung im Nebenstrom (3) ermöglichen,
wobei die Umgehungsschleifenanordnung im Nebenstrom (3) eine Qualitätsmessungszelle (12) und ein Magnetventil (13) umfasst,
und die Umgehungsschleifenanordnung im Nebenstrom (3) Sensoren (11) zur Messung wenigstens eines der Parameter Trübung, Leitfähigkeit, pH-Wert, Ultraschall und mikrobiologischer Aktivität umfasst.

6. Vorrichtung (1) gemäß Anspruch 5, wobei der Messtank (12) an einer Umgehungsleitung (14) innerhalb der Umgehungsschleifenanordnung im Nebenstrom (3) angeordnet ist und wobei die Umgehungsschleifenanordnung im Nebenstrom (3) ferner eine Ejektorpumpe (15) umfasst.

7. Vorrichtung (1) gemäß einem der Ansprüche 5-6, wobei die Umgehungsschleifenanordnung im Nebenstrom (3) als eine Absaugeinheit an der Niederdruckseite der zum Rückführen von Wasser vorgesehenen Vorrichtung (1) angeordnet ist.

8. Vorrichtung (1) gemäß einem der Ansprüche 5-7, wobei die Umgehungsschleifenanordnung im Nebenstrom (3) einen Ultraschallwandler (30) umfasst.

9. Vorrichtung (1) gemäß einem der Ansprüche 5-8, wobei die Umgehungsschleifenanordnung im Nebenstrom (3) eine Trübungssensoreinheit umfasst.

## Revendications

1. Procédé destiné à mesurer un ou plusieurs paramètres dans un écoulement d'eau dans un dispositif (1) sous la forme d'une douche à recirculation, ledit dispositif (1) comprenant une entrée d'eau douce (5) pour l'eau froide et chaude, qui rencontre une boucle de recirculation comprenant une entrée d'eau de recirculation (6), une voie de passage (4) pour l'eau recyclée et une sortie d'eau de recirculation (7), une sortie utilisateur (8) sous la forme d'un pommeau de douche, un réchauffeur (9) et un filtre (10) disposés le long de la boucle de recirculation, et dans lequel le dispositif (1) comprend également un agencement de boucle de contournement hors ligne (3) qui comprend un espace de stagnation de liquide (2), lequel agencement de boucle de contournement hors ligne (3) comprend également un ou plusieurs capteurs (11) permettant des mesures dans l'agencement de boucle de contournement hors ligne (3),
ledit procédé comprenant l'acheminement d'une partie de l'écoulement d'eau jusqu'à l'espace de stagnation de liquide (2) où la mesure est effectuée,
dans lequel l'emplacement de stagnation de liquide (2) est agencé comme un agencement de boucle de contournement hors ligne (3) comprenant une cellule de mesure de qualité (12) dans le dispositif (1) destiné au recyclage de l'eau,
et dans lequel la mesure est effectuée sur un ou plusieurs des paramètres suivants : turbidité, conductivité, valeur de pH, ultrasons et activité microbiologique.

2. Procédé selon la revendication 1, dans lequel l'étape d'acheminement d'une partie de l'écoulement d'eau jusqu'à l'espace de stagnation de liquide (2) est effectuée de façon cyclique.

3. Procédé selon l'une quelconque des revendications précédentes, dans lequel la mesure est effectuée au moins sur la turbidité.

4. Procédé selon l'une quelconque des revendications précédentes, dans lequel la mesure est effectuée sur la turbidité, dans lequel une valeur de référence est définie comme valeur de départ par mesure de la lumière pénétrant dans l'eau douce, et celle-ci est utilisée comme référence, et dans lequel la turbidité est ensuite mesurée par comparaison de la pénétration de la lumière dans le liquide avec la référence, et en tant que telle permet de déterminer la qualité de l'eau.

5. Dispositif (1) sous la forme d'une douche à recirculation, ledit dispositif (1) comprenant une voie de passage (4) pour l'eau recyclée, une entrée d'eau douce (5) pour l'eau froide et chaude, qui rencontre une boucle de recirculation comprenant une entrée d'eau de recirculation (6), une voie de passage (4) pour l'eau recyclée et une sortie d'eau de recirculation (7), une sortie utilisateur (8) sous la forme d'un pommeau de douche, un réchauffeur (9) et un filtre (10) disposés le long de la boucle de recirculation, le dispositif (1) étant **caractérisé en ce qu'**il comprend également un agencement de boucle de contournement hors ligne (3) qui comprend un espace de stagnation de liquide (2), lequel agencement de boucle de contournement hors ligne (3) comprend également un ou plusieurs capteurs (11) permettant des mesures dans l'agencement de boucle de contournement hors ligne (3),
dans lequel l'agencement de boucle de contournement hors ligne (3) comprend une cellule de mesure de qualité (12) et une vanne magnétique (13),
et dans lequel l'agencement de boucle de contournement hors ligne (3) comprend des capteurs (11) pour la mesure d'au moins un des paramètres suivants : turbidité, conductivité, valeur de pH, ultrasons et activité microbiologique.

6. Dispositif (1) selon la revendication 5, dans lequel le réservoir de mesure (12) est disposé sur une conduite de contournement (14) à l'intérieur de l'agencement de boucle de contournement hors ligne (3), et dans lequel l'agencement de boucle de contournement hors ligne (3) comprend également une pompe d'éjection (15).

7. Dispositif (1) selon l'une quelconque des revendications 5 et 6, dans lequel l'agencement de boucle de contournement hors ligne (3) est agencé comme une unité d'aspiration sur le côté basse pression du dispositif (1) destiné au recyclage de l'eau.

8. Dispositif (1) selon l'une quelconque des revendications 5 à 7, dans lequel l'agencement de boucle de contournement hors ligne (3) comprend un transducteur ultrasonore (30).

9. Dispositif (1) selon l'une quelconque des revendications 5 à 8, dans lequel l'agencement de boucle de contournement hors ligne (3) comprend une unité de capteur de turbidité.
